# FASCICULE DE BREVET EUROPEEN

(11) **EP 0 645 139 B1**
(45) Date de publication et mention de la délivrance du brevet: **19.04.2000**
(21) Numéro de dépôt: 94870152.9
(22) Date de dépôt: 21.09.1994
(51) Int. Cl.: A61K 31/40

(54) **Utilisation du (S)-(-)-alpha-éthyl-2-oxo-1-pyrrolidineacétamide pour la préparation de médicaments destinés au traitement de l'anxiété**
Verwendung von (S)-(-)-alpha-ethyl-2-oxo-1-pyrrolidinacetamid zur Herstellung eines Medikaments zur Behandlung von Angst
Use of (S)-(-)-alpha-ethyl-2-oxo-1-pyrrolidine-acetamide for the preparation of a medicament for the treatment of anxiety

(30) Priorité: 24.09.1993 GB 9319732
(43) Date de publication de la demande: 29.03.1995
(73) Titulaire: UCB, S.A., 1070 Bruxelles (BE)
(72) Inventeur: Wülfert, Ernst, B-1180 Bruxelles (BE); Gobert, Jean, B-1040 Bruxelles (BE); Gower, Alma, B-1420 Braine-L'Alleud (BE); Cossement, Eric, B-1050 Bruxelles (BE)
(74) Mandataire: Dusseldorp, Raymond

(56) Documents cités:
- EP-A- 0 162 036
- EUR J PHARMACOL (NETHERLANDS), NOV 10 1992, VOL. 222, NO. 2-3, PAGE(S) 193-203, Gower AJ et al 'ucb L059, a novel anti-convulsant drug: pharmacological profile in animals [published erratum appears in Eur J Pharmacol 1993 Jan 19;230(3):389]'
- EUR J PHARMACOL (NETHERLANDS), MAR 2 1993, VOL. 232, NO. 2-3, PAGE(S) 147-58, Loscher W et al 'Profile of ucb L059, a novel anticonvulsant drug, in models of partial and generalized epilepsy in mice and rats.'
- PSYCHOPHARMACOL BULL (UNITED STATES), 1989, VOL. 25, NO. 3, PAGE(S) 498-502, Wulfert E et al 'Facilitation of calcium-dependent cholinergic function by ucb L059, a new'
- J AFFECT DISORD (NETHERLANDS), DEC 1979, VOL. 1, NO. 4, PAGE(S) 227-35, File SE et al 'Evidence that piracetam has an anxiolytic action.'
- POSCHEL BPH, MARRIOTT JG, GLUCKMAN MI. Pharmcology of the cognition activator pramiracetam (Cl-879). DRUGS EXPTL. CLIN. RES. , Volume IX(12), 1983, pages 853-871.
- PETKOV VD, GETOVA D, MOSHARROF AH. A Study of Nootropic drugs for anti-anxiety action. ACTA PHYSIOLAGICA ET PHARMACOLOGICA BULGARICA, Volume 13, No. 4, 1987, pages 25-30.
- NOOTROPYL (piracetam) monography, Vidal 1993.
- NOOTROPYL (piracetam) monography, Vidal 1997.
- Goodman and Gilman's "The Pharmacological Basis of Therapeutics" 9th Ed (1996), McGraw-Hill, Ch 18 : Drugs used in the treatment of anxiety ; pages 420-427.

## Description

La présente invention se rapporte à l'utilisation du (S)-(-)-á-éthyl-2-oxo-1-pyrrolidineacétamide pour la préparation de médicaments destinés au traitement de l'anxiété.

L'utilisation du (S)-á-éthyl-2-oxo-1-pyrrolidineacétamide lévogyre en tant qu'agent protecteur pour le traitement et la prévention des agressions du type hypoxique et ischémique du système nerveux central est décrite dans le brevet européen 162.036 au nom de la demanderesse. Ce composé trouve également une application dans le traitement de l'épilepsie, indication thérapeutique pour laquelle on a démontré que son énantiomère dextrogyre, le (R)-(+)-á-éthyl-2-oxo-1-pyrrolidineacétamide est totalement dépourvu d'activité (A.J.GOWER et al., Eur.J.Pharmacol.,222,(1992),193-203). L'utilisation du (S)-á-éthyl-2-oxo-1-pyrrolidineacétamide lévogyre pour le traitement de l'anxiété n'est toutefois pas connue.

Ce composé, qui est un dérivé proche du Piracetam, potentialise de façon stéréosélective les fonctions cholinergiques (Psychopharmacol. Bull., 1989, 25(3), pages 498-502), celui-ci est un agent nootropique beaucoup plus actif que le Piracetam.

D'après la publication (J. Affect. Disord., 1979, 1 (4), pages 227-235), le Piracetam semble avoir une action anxiolytique, l'hypothèse selon laquelle l'effet cognitif du Piracetam serait dû à ses propriétés anxiolytiques, est proposée. Pourtant d'autres publications (Drugs Exptl. Clin. Res. IX(12), 1983, pages 853-871 et Acta Physiologica et Pharmacologica Bulgaria, 1987, 13(4), pages 25-39), montrent qu'au contraire, le Piracetam n'induit pas d'effet anxiolytique.

Dans le brevet européen 162.036 mentionné plus haut, on décrit aussi des procédés de préparation du (S)-(-)-á-éthyl-2-oxo-1-pyrrolidineacétamide qui nécessitent la synthèse d'un réactif de départ obtenu par dédoublement du composé racémique correspondant. Dans le brevet britannique 2.225.322, également au nom de la demanderesse, on décrit un procédé de préparation de ce composé qui offre l'avantage d'utiliser comme matière première un acide aminé naturel ayant déjà la configuration stéréochimique souhaitée, ce qui permet d'éviter la séparation fastidieuse des énantiomères.

Poursuivant ses travaux de recherche sur ce composé, la demanderesse a trouvé maintenant que le (S)-(-)-á-éthyl-2-oxo-1-pyrrolidineacétamide possède des propriétés anxiolytiques ayant un intérêt thérapeutique notable.

De plus, cette activité anxiolytique n'a pas pu être retrouvée avec l'énantiomère dextrogyre, le (R)-(+)-á-éthyl-2-oxo-1-pyrrolidineacétamide.

La présente invention a donc pour objet l'utilisation du (S)-(-)-á-éthyl-2-oxo-1-pyrrolidineacétamide de formule 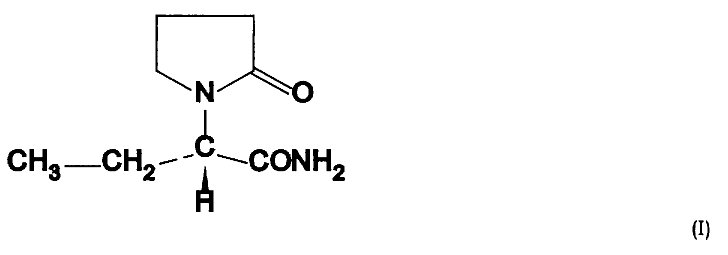 pour la préparation de médicaments destinés au traitement de l'anxiété .

L'activité anxiolytique du (S)-(-)-α-éthyl-2-oxo-1-pyrrolidineacétamide est plus particulièrement marquée dans des situations pharmacologiques dans lesquelles l'état émotionnel initial a été exacerbé, par exemple par le choix d'animaux particulièrement sensibles ou âgés, ou par le choix de conditions expérimentales faisant appel à l'anticipation d'un stimulus répulsif.

La dépendance entre l'activité anxiolytique du composé de formule I et l'intensité de l'état émotionnel initial laisse présager que l'application thérapeutique de ce composé sera de préférence orientée vers le traitement des états d'anxiété pathologique. Cette sélectivité distingue nettement le composé utilisé conformément à l'invention des médicaments anxiolytiques existants du type benzodiazépine, et présente un avantage important sur ces autres classes de produits qui agissent indistinctement sur un animal anxieux ou sur un animal normal. Dans ce dernier cas, l'activité anxiolytique s'accompagne d'une désinhibition du comportement général normal qui induit chez le sujet sain une réponse adaptative inadéquate qu'il est souhaitable d'éviter. Dans ce contexte, le (S)-(-)-α-éthyl-2-oxo-1-pyrrolidineacétamide facilite la récupération et le retour à une situation plus normale dans des états d'anxiété pathologique dus à des troubles d'origine neuroendocrinienne produits par un stress chez les sujets âgés par opposition aux benzodiazépines qui ne favorisent pas cette récupération. Contrairement aux benzodiazépines dont l'amnésie et les perturbations neuromotrices telles que l'ataxie, la relaxation musculaire et la sédation constituent des effets secondaires bien connus et indésirables (J.H.WOODS et al., Pharmacol.Rev., 39, (1987), 251-419), le (S)-(-)-α-éthyl-2-oxo-1-pyrrolidineacétamide aux doses thérapeutiques utilisées, ne produit pas le moindre effet nuisible sur la mémoire et ne provoque pas d'effets neuromoteurs gênants. Une grande marge de sécurité existe en effet entre les doses anxiolytiques et les doses neurotoxiques ou sédatives chez l'animal. (A.J. GOWER et al., loc.cit.).

En outre, le (S)-(-)-α-éthyl-2-oxo-1-pyrrolidineacétamide supprime également l'anxiété induite par le sevrage d'une administration chronique de benzodiazépines.

Il résulte de cet éventail imprévisible de propriétés que l'utilisation du (S)-(-)-α-éthyl-2-oxo-1-pyrrolidineacétamide est particulièrement intéressante pour la préparation de médicaments destinés au traitement des états d'anxiété, tels que plus spécifiquement l'anxiété généralisée, la panique, l'agoraphobie, la phobie sociale, les troubles obsessionnels, l'anxiété due à un stress aigu post-traumatique, le sentiment de danger menaçant, l'absence de tonus, la peur et la tension dont les manifestations parfois concomitantes d'ordre physiologique sont la tachycardie, la dyspnée, la transpiration, le tremblement, la faiblesse et la fatigue. (International Statistical Classification of Diseases and Related Health Problems - Tenth Revision: Volume 1. World Health Organization, Geneva, 1992).

La présente invention requiert l'administration d'une dose efficace du composé de formule I pour traiter l'anxiété. La dose nécessaire selon la présente invention sera suffisamment élevée pour permettre le soulagement de l'anxiété. Les compositions pharmaceutiques renfermant le composé de formule I peuvent être administrées par exemple, par voie orale ou parentérale, c'est-à-dire intraveineuse, intramusculaire et sous-cutanée.

Les compositions pharmaceutiques utilisables pour l'administration orale peuvent être solides ou liquides, et se présentent par exemple sous la forme de comprimés, pilules, dragées, capsules en gélatine, solutions, sirops, etc...

Dans ce but, le composé actif peut être utilisé en mélange avec un diluant inerte ou un véhicule non toxique pharmaceutiquement acceptable tel que par exemple de l'amidon ou du lactose. Eventuellement, ces compositions pharmaceutiques peuvent aussi contenir un agent liant tel que la cellulose microcristalline, la gomme adragante ou la gélatine, un agent désintégrant tel que l'acide alginique, un lubrifiant tel que le stéarate de magnésium, un agent de coulance tel que le dioxyde de silicium colloïdal, un édulcorant comme le sucrose ou la saccharine, des colorants ou un aromatisant comme la menthe poivrée ou le salicylate de méthyle. Elles comprennent également les compositions qui permettent de délivrer la substance active de manière progressive.

Les compositions pharmaceutiques utilisables pour l'administration parentérale sont les formes pharmaceutiquement connues pour ce genre d'administration et se présentent sous la forme de solutions ou suspensions aqueuses ou huileuses généralement contenues dans des ampoules, des seringues à usage unique, des fioles en verre ou en matière plastique, ou des récipients de perfusion.

Outre le composé actif, ces solutions ou suspensions peuvent encore contenir éventuellement un diluant stérile tel que l'eau pour injection, du sérum physiologique, des huiles, des polyéthylène glycols, de la glycérine, du propylène glycol ou d'autres solvants synthétiques, des agents antibactériens tels que l'alcool benzylique, des antioxydants comme l'acide ascorbique ou le bisulfite de sodium, des chélatants comme l'acide éthylènediamine-tétraacétique, des tampons tels que les acétates, citrates ou phosphates et des agents pour ajuster l'osmolalité comme le chlorure de sodium ou le dextrose.

Ces formes pharmaceutiques sont préparées selon les méthodes couramment utilisées par les pharmaciens.

Le pourcentage de produit actif dans les compositions pharmaceutiques peut varier dans des limites très larges de concentration et dépend d'une variété de facteurs tels que le sexe du patient, son âge, son poids et sa condition médicale ainsi que du mode d'administration. Ainsi la quantité de matière active dans les compositions destinées à l'administration orale est au moins de 0,5% en poids et peut atteindre jusque 80 % en poids par rapport au poids de la composition. Dans les compositions orales préférées, la dose unitaire est comprise entre 50 et 1000 mg de produit actif.

Dans les compositions destinées à l'administration par la voie parentérale, la quantité de matière active présente est au moins de 0,5% en poids et peut atteindre 33% en poids par rapport au poids de la composition. Dans les compositions parentérales préférées, la dose unitaire est comprise entre 1 mg et 200 mg de produit actif.

Quant à la posologie journalière, elle peut varier dans une gamme étendue de doses unitaires de produit actif, et est généralement comprise entre 5 et 70 mg/kg. Une dose moyenne de 250 mg, deux fois par jour, s'est révélée efficace chez l'homme pour soulager l'anxiété. Il est entendu cependant que des dosages spécifiques peuvent être adaptés à des cas particuliers en fonction des besoins individuels laissés à l'appréciation du médecin. Les dosages évoqués ci-dessus sont donnés à titre indicatif et ne limitent en aucune manière la façon de mettre l'invention en pratique.

A titre d'exemple non limitatif d'une composition contenant le (S)-(-)-α-éthyl-2-oxo-1-pyrrolidineacétamide pouvant être administrée par voie orale, on donne ci-après quatre formulations pour gélules de gélatine blanches et opaques:

| | Numéro des gélules | | | |
|---|---|---|---|---|
| | 1 | 1 | 1 | 0 |
| Composé I | 62,5 mg | 125 mg | 250 mg | 500 mg |
| Lactose | 362,5 mg | 264 mg | 89 mg | 50 mg |
| Stéarate de magnésium | 1,0 mg | 1 mg | 1 mg | 2 mg |

L'efficacité du (S)-(-)-α-éthyl-2-oxo-1-pyrrolidineacétamide pour le traitement de l'anxiété est démontrée par son activité dans les essais pharmacologiques suivants effectués sur animaux au moyen de tests standards reconnus pour leur capacité à mettre en évidence l'activité anxiolytique de nouveaux composés.

L'anxiété peut exister sous différentes formes physiologiques et pathologiques. L'hétérogénéité des troubles de l'anxiété est cliniquement acceptée comme il est accepté également que les différents tests d'anxiété pratiqués sur animaux, basés sur des modifications du comportement, sont sensibles à des états d'anxiété différents. (S.E.FILE, "Animal Models of anxiety" dans Biological Psychiatry, Vol.2, Eds. G.Racagui et al., Excerpta Medica, Amsterdam, (1991), p.596-599).

Cependant, pour être assuré qu'un test de comportement donné permet bien de détecter une activité anxiolytique, celle-ci demande encore à être confirmée par des essais cliniques. Un essai chez l'homme a permis de confirmer l'efficacité thérapeutique du (S)-(-)-α-éthyl-2-oxo-1-pyrrolidineacétamide dans le traitement de divers états d'anxiété.
1.- Test de la planche à trous. (Exploration).
La "planche à trous" offre une méthode simple pour étudier le comportement d'un rongeur et pour mesurer la réaction de l'animal lorsque celui-ci est placé dans un environnement non familier. Cette réaction, dite "réaction d'exploration" est en rapport à la fois avec la curiosité et avec le désir de fuite de l'animal et est influencée par des médicaments psychoactifs.
Cette méthode a notamment été utilisée pour prévoir l'activité anxiolytique potentielle des benzodiazépines (N.A.NOLAN et M.W.PARKES, Psychopharmacologia (Berl.),29, (1973),277-288). Utilisant la méthodologie de J.R. BOISSIER et P. SIMON (Arch.Int.Pharmacodyn. 147,(1964),372-387), le test consiste à placer une souris au centre d'une planche carrée percée de 16 trous régulièrement espacés et à compter le nombre de fois que l'animal plonge la tête dans un trou pendant une période d'exploration de 5 minutes. Trois souches de souris génétiquement distinctes ont été utilisées dans ce test: une souche normale de souris NMRI et deux souches de souris émotivement plus sensibles, l'une prédisposée à la crise audiogène (Dilute Brown Agouti-derived (DBA-derived)), et l'autre dont le réflexe normal de fuite est bloqué par la peur (souche C57 Black mice).
Les activités du (S)-(-)-α-éthyl-2-oxo-1-pyrrolidineacétamide (composé I), de son énantiomère dextrogyre, le (R)-(+)-α-éthyl-2-oxo-1-pyrrolidineacétamide (composé II) et du diazépam ont été comparées sur ces trois souches.
Les composés sont administrés à l'animal par injection intrapéritonéale (10 ml/kg de souris) 30 minutes avant l'exposition à la planche. Les animaux des groupes témoins reçoivent le véhicule.
Le tableau I indique la moyenne des nombres de trous explorés par groupe de 16 animaux (X ± SEM) pour chacune des souches (animaux témoins et traités) aux doses indiquées (SEM : Déviation standard sur la moyenne). Il donne aussi la variation du score exprimée en pour-cent par rapport au score des groupes témoins. 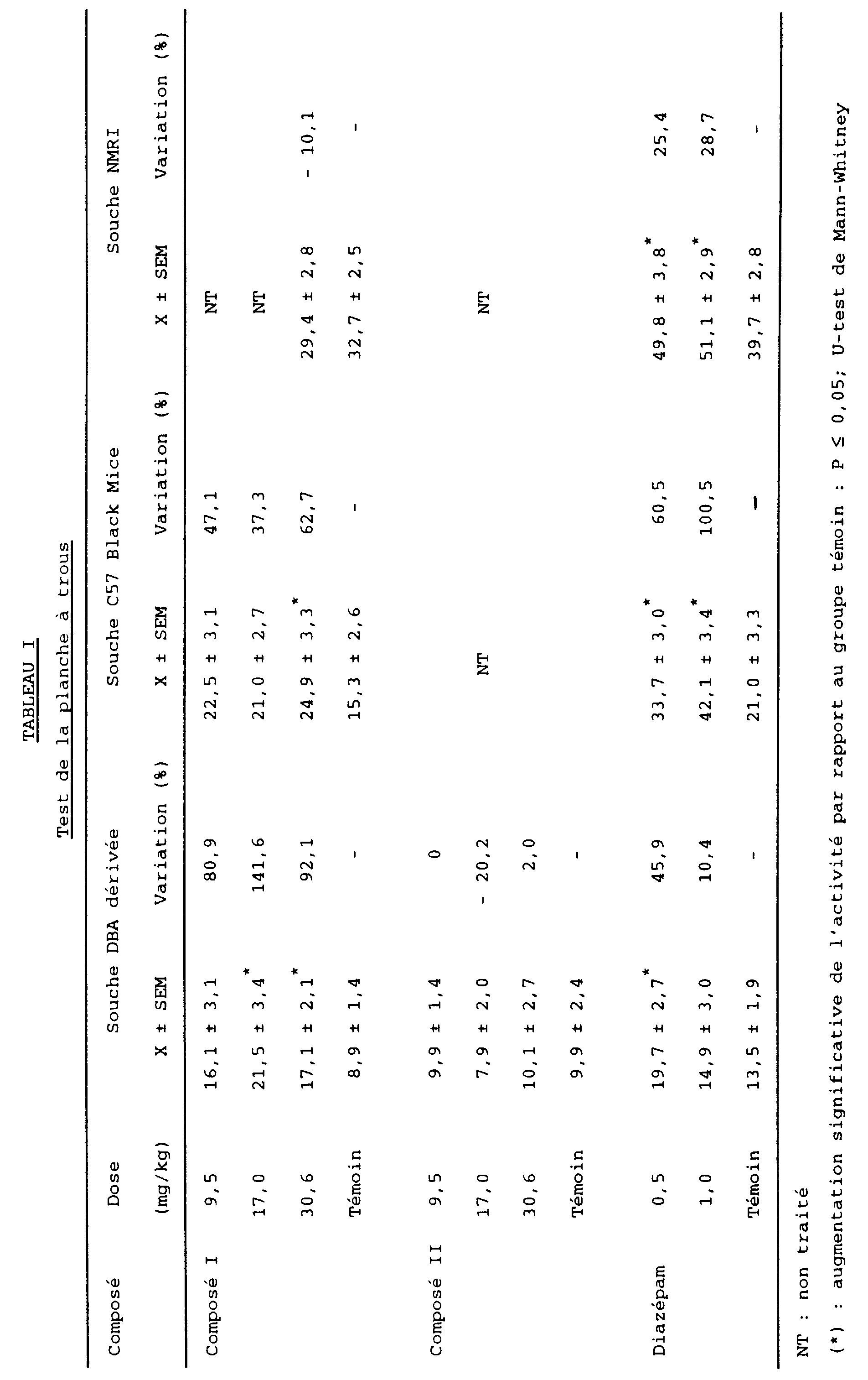
Les résultats montrent que les trois souches de souris se distinguent nettement par le score de base (témoins): le score étant beaucoup plus élevé chez la souris normale NMRI que chez les deux autres souches. Il est connu que le nombre de trous explorés est fortement réduit sous l'influence d'agents anxiogènes, comme par exemple la caféine ou la yohimbine (R.LISTER., Pharmacol. Ther., 46, (1990), 321-340). Par conséquent, le faible score observé pour les groupes témoins des souches maladives DBA-dérivée et C57 Black Mice reflète correctement le taux élevé de l'anxiété intrinsèque de ces deux souches.
Comme le diazépam, le (S)-(-)-α-éthyl-2-oxo-1-pyrrolidineacétamide augmente considérablement le score des souches DBA-dérivée et C57 Black Mice, mais, contrairement au diazépam, le composé I n'a pratiquement pas d'effet anxiolytique sur la souche normale NMRI. Sur cette dernière souche, le diazépam est également bien actif et l'augmentation du score traduit bien l'effet de désinhibition propre aux benzodiazépines.
La dépendance de l'effet anxiolytique du composé I par rapport à la nature de la souche, et la sélectivité marquée de cet effet sur des souches qui présentent une anxiété maladive suggère que le composé I est particulièrement indiqué pour traiter plus spécifiquement les états émotionnels exacerbés, indépendamment de tout effet désinhibiteur du comportement. L'énantiomère (R)-(+)-α-éthyl-2-oxo-1-pyrrolidineacétamide (composé II) est inactif sur ces souches.
2.- Test des quatre plaques (comportement puni)
L'approche générale utilisée dans ce type d'expérience consiste à provoquer l'inhibition d'une réponse spécifique au moyen d'un stimulus qui génère une aversion au moment de cette réponse spécifique.
Le test des "quatre plaques" est une méthode facile décrite pour la première fois par J.R.BOISSIER et al., dans Eur.J.Pharmacol., 4,(1968),145-151, pour évaluer l'activité anxiolytique potentielle de nouveaux composés sur des animaux de laboratoire. Il consiste, dans une première situation, à placer une souris dans un environnement qui ne lui est pas familier, constitué par une surface couverte de quatre plaques métalliques qui peuvent être électrifiées, et à compter le nombre de fois que l'animal traverse la surface en passant d'une plaque à l'autre (traversées impunies) pendant un temps déterminé. Dans une seconde situation, l'animal est puni par un choc électrique dans les pattes chaque fois qu'il traverse la surface de part en part (traversées punies) ce qui a pour effet de provoquer chez l'animal une réaction d'immobilisation. Dans les conditions où il y a punition (traversées punies), le nombre de traversées de la surface pour explorer l'environnement est fortement réduit. Par contre, cette diminution du nombre de traversées dans les conditions où il y a punition est inhibée lorsque l'animal a été traité au préalable avec un agent anxiolytique.
Les composés testés, le (S)-(-)-α-éthyl-2-oxo-1-pyrrolidineacétamide (composé I) et le chlordiazépoxide, sont administrés à l'animal par injection intrapéritonéale (10 ml/kg de souris) 30 minutes avant le début du test. Les animaux des groupes témoins reçoivent seulement le véhicule. Le tableau II donne la moyenne (X ± SEM) des nombres de traversées dans les conditions punies et dans les conditions impunies pour des groupes de 30 souris NMRI, pendant une période de 1 minute, aux doses indiquées.
Il donne également la variation du score exprimée en pour-cent par rapport au score des groupes témoins. 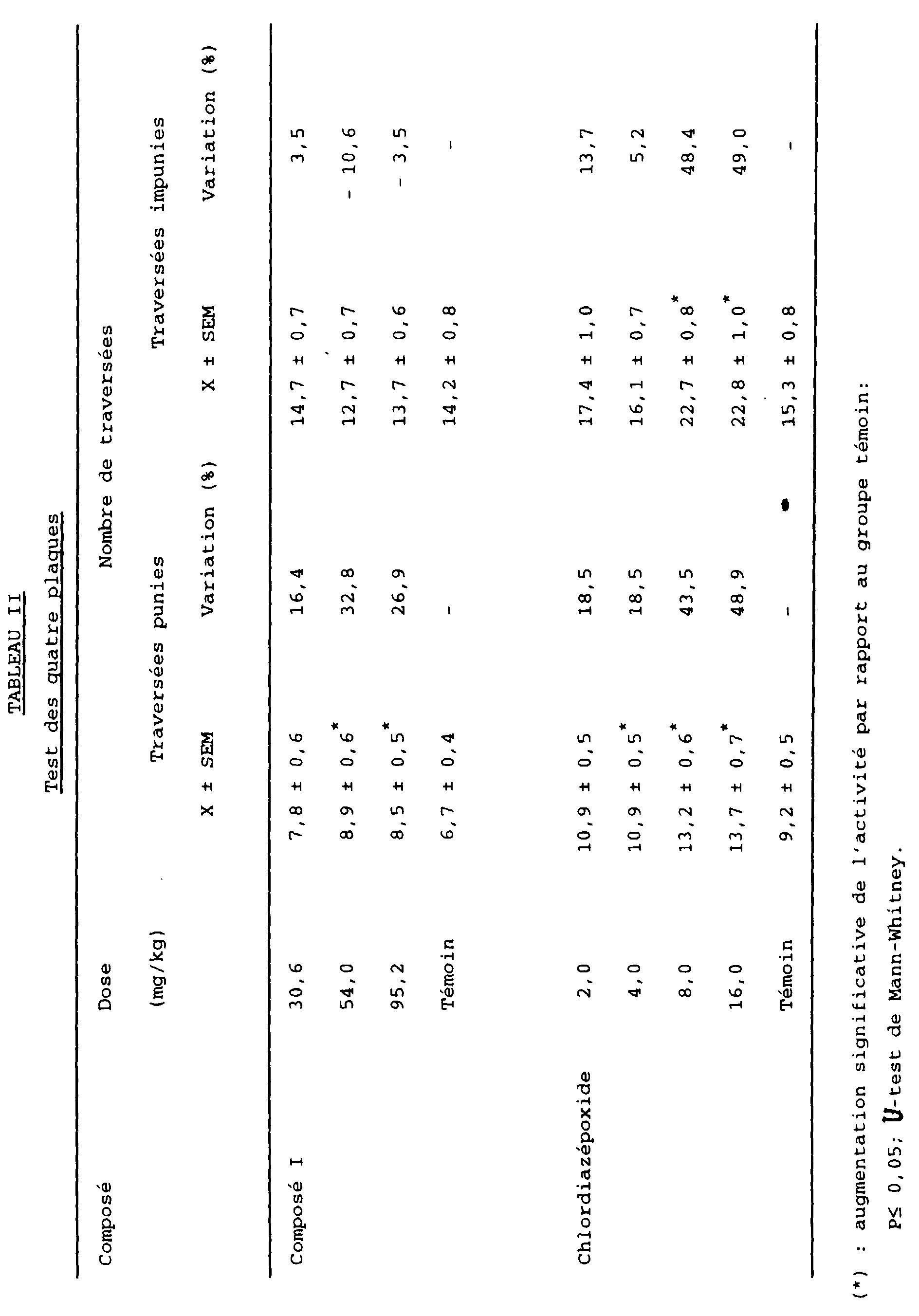
Les résultats montrent que dans les conditions punies, le nombre de traversées est moins élevé que dans les conditions impunies à la fois pour les groupes témoins et pour les groupes traités.
Le composé I et le chlordiazépoxide augmentent le nombre de traversées dans les conditions punies par rapport aux groupes témoins, mais contrairement au composé I, le chlordiazépoxide exerce également le même effet dans les conditions impunies, par désinhibition du comportement normal.
Ces résultats montrent que le (S)-(-)-α-éthyl-2-oxo-1-pyrrolidineacétamide (composé I) offre un avantage sur les médicaments existants par le fait qu'il manifeste seulement son activité anxiolytique dans des situations qui induisent une forte anxiété et non pas dans des situations qui sont normales.
Cette sélectivité du composé I permet de distinguer un effet anxiolytique d'un effet qui aurait pour résultat l'augmentation de l'activité per se.
3.- Test du labyrinthe en croix surélevé.
Ce test constitue une méthode simple et rapide, largement utilisée pour détecter l'activité anxiolytique de nouveaux composés. Contrairement à la plupart des tests d'anxiété qui font appel à des stimuli nociceptifs, par exemple un choc électrique, cet essai s'appuie uniquement sur la mesure de l'activité spontanée de l'animal confronté à une situation anxiogène naturelle, génératrice d'un conflit entre deux tendances opposées: celle d'explorer un nouvel environnement et celle de fuir un espace surélevé et ouvert. L'exploration relative des bras ouverts ou fermés du labyrinthe est un reflet de l'anxiété de l'animal, l'exploration des bras ouverts étant fortement réduite chez les animaux présentant un état d'anxiété élevé (S.PELLOW et al., J.Neurosci. Methods, 14, (1985), 149-167).
Un composé anxiolytique augmente le nombre de visites des bras ouverts et le temps consacré à les explorer, bien que dans le cas des benzodiazépines, une part de cette augmentation ait été attribuée à l'induction d'une stéréotypie par le médicament aux doses indiquées (U.FALTER et al., Behav. Processes,29, (1993),128-129).
La technique utilisée dans ce test est celle décrite par S.PELLOW (loc.cit.) modifiée par R.J. RODGERS et al. (Psychopharmacology, 106, (1992), 102-110). Cette modification consiste à augmenter l'état d'anxiété de l'animal en le plaçant au préalable sur un labyrinthe surélevé (prétest) dont les quatres bras sont ouverts, ce qui a pour effet de diminuer le nombre d'explorations des bras ouverts (score de base) lorsque l'animal est placé 24 heures plus tard sur un labyrinthe classique à 2 bras ouverts et 2 bras fermés.
Les activités du (S)-(-)-α-éthyl-2-oxo-1-pyrrolidineacétamide (composé I), de l'énantiomère dextrogyre (composé II) et du chlordiazépoxide ont été examinées dans ce test. Les composés sont administrés à des rats Sprague-Dawley par voie intrapéritonéale (10 ml/kg de rat) 60 minutes avant le début du test. Les animaux des groupes témoins reçoivent une solution saline (0,9 % NaCl).
Le tableau III donne les moyennes du nombre total d'entrées dans les bras du labyrinthe, du pourcentage d'entrées dans les bras ouverts par rapport au nombre total d'entrées, et du temps consacré à l'exploration de ces bras ouverts, pour des groupes de 15 rats, témoins ou traités aux doses indiquées, pendant 4 minutes.

**TABLEAU III**

| Test du labyrinthe en croix surélevé | | | | |
|---|---|---|---|---|
| Composés | Dose (mg/kg) | Nombre total d'entrées X ± SEM | Bras ouverts | |
| | | | % entrées X ± SEM | temps (sec) X ± SEM |
| Témoin | | 8,6 ± 1,0 | 10,3 ± 3,0 | 5,2 ± 2,0 |
| Composé I | 17,0 | 11,9 ± 0,7* | 20,2 ± 3,0* | 16,1 ± 3,1* |
| Composé II | 17,0 | 8,3 ± 0,9 | 10,9 ± 3,1 | 5,7 ± 2,2 |
| | 54,0 | 9,2 ± 0,7 | 11,8 ± 2,7 | 6,8 ± 1,6 |
| Chlordiazépoxide | 5,0 | 15,0 ± 1,3* | 27,1 ± 2,7* | 27,1 ± 3,7* |

| | | | | |
|---|---|---|---|---|
| (*) : augmentation significative de l'activité par rapport au groupe témoin: P ≤ 0,05; U-test de Mann-Whitney. | | | | |

Les résultats montrent que pour le groupe témoin, le nombre d'entrées dans les bras ouverts ne représente qu'un très faible pourcentage du nombre total des entrées.
Le traitement des animaux par le composé I ou par le chlordiazépoxide augmente significativement le nombre des entrées dans les bras ouverts ainsi que le temps qui est consacré à les explorer. Par contre, le composé II n'a pas d'activité.
Ces résultats confirment l'intérêt du composé I pour traiter les états d'anxiété pathologique.
4.- Test du saisissement potentialisé.
La réaction de saisissement à un bruit violent (agression sonore) est potentialisée par la présentation simultanée d'un stimulus lumineux lorsque au préalable, ce stimulus lumineux a toujours été accompagné d'un choc électrique dans les pattes de l'animal. Dans ce cas, l'anxiété induite par l'agression combinée du son et de la lumière (saisissement potentialisé) provient de la prémonition d'un événement douloureux et désagréable (M.DAVIS, Psychopharmacology, 62, (1979), 1-7).
Les composés anxiolytiques, tels que les benzodiazépines ou la buspirone par exemple, réduisent l'amplitude de la réaction de saisissement potentialisé proportionnellement à la dose utilisée (S.GREEN et al., "Animal Models of Anxiety" dans Behavioural Models in Psychopharmacology. Ed.P.Willner, Cambridge Univ.Press, (1991), 21-49; M.DAVIS, Trends Pharmacol.Sci., 13, (1992), 35-41).
La technique utilisée dans ce test est inspirée de celle de M.DAVIS (loc.cit.) et comprend essentiellement deux phases:
- 1^{ère} phase:: entraînement - les animaux sont entraînés à réagir à un stimulus lumineux accompagné d'un choc électrique dans les pattes;
- 2^{ème} phase:: test principal - on mesure l'amplitude de la réaction de saisissement des animaux à une agression sonore accompagnée d'un stimulus lumineux sans choc électrique - 20 essais - (réponse de saisissement potentialisé ou RSP) et on mesure également l'amplitude de la réaction de saisissement des animaux à une agression sonore non accompagnée d'un stimulus lumineux ni du choc électrique - 20 essais - (réponse de saisissement acoustique ou RSA).

On utilise des groupes de 10 rats mâles Sprague-Dawley naïfs.
Les composés testés sont le (S)-(-)-α-éthyl-2-oxo-1-pyrrolidineacétamide (composé I) et le chlordiazépoxide. Ils sont administrés par voie intrapéritonéale (1 ml/kg de rat) 60 minutes avant le test principal. Les animaux du groupe témoin reçoivent seulement la solution saline (0,9 % NaCl).
Au moment du test, chaque animal est placé dans une cage reliée à des accéléromètres qui enregistrent automatiquement les sauts de l'animal et expriment l'amplitude de la réponse en unités arbitraires.
Le tableau IV donne les moyennes des amplitudes obtenues pour les réponses RSA et RSP aux doses indiquées.

**TABLEAU IV**

| Test du saisissement potentialisé | | | |
|---|---|---|---|
| Composé | Dose en mg/kg | RSP X ± SEM | RSA X ± SEM |
| Témoin | | 22965 ± 4760* | 17326 ± 3126 |
| Composé I | 17,0 | 14817 ± 3056** | 14388 ± 2214 |
| Chlordiazépoxide | 5,0 | 10393 ± 2079** | 8759 ± 2352** |

| | | | |
|---|---|---|---|
| (*): Différence significative entre RSP et RSA: P ≤ 0,05, test t pairé | | | |
| (**): Différence entre groupe traité et groupe témoin significative: P ≤ 0,05; test t de Student. | | | |

Les résultats montrent que dans le groupe des animaux témoins, la réponse RSP est plus intense que la réponse RSA comme prévu. Le composé I réduit fortement l'amplitude de la réponse de saisissement potentialisé (RSP) mais n'a que peu d'effet sur la réponse de saisissement acoustique (RSA).
Le chlordiazépoxide, par contre, atténue l'amplitude des deux réponses de manière significative. L'influence des benzodiazépines sur la RSA illustre un inconvénient inhérent à cette classe de composés et qui est attribuable à leur effet sédatif.
Un composé tel que le (S)-(-)-(α-éthyl-2-oxo-1-pyrrolidineacétamide qui agit spécifiquement sur la réponse RSP peut être mieux indiqué pour traiter spécifiquement les états d'anxiété pathologique et offre par conséquent un avantage sur les thérapies existantes.
5.- Réponse neuroendocrinienne à un stress.
Les réponses adaptatives à un stress, faisant intervenir des mécanismes de rétro-contrôle dans le système hypothalamique-pituitaire-adrénergique, sont profondément altérées chez le rat âgé. Ces altérations dues à l'âge peuvent être mises en évidence par la mesure du taux basal de corticostérone plasmatique qui est beaucoup plus élevé chez les sujets âgés que chez les sujets jeunes (R.M.SAPOLSKY, Neurobiol.Aging, 13, (1991), 171-174).
Une situation de stress fait rapidement augmenter le taux de corticostérone plasmatique aussi bien chez les animaux jeunes que chez les animaux âgés, mais ces derniers, après le stress, récupèrent beaucoup plus lentement et de manière moins complète (R.M.SAPOLSKY et al., Exp.Gerontol.,18, (1983), 55-64; A.M.ISSA et al.,J.Neurosci., 10, (1990), 3247-3254).
Les conséquences de ces effets liés à l'âge sont importantes par le fait que, s'il est vrai que les glucocorticoïdes sont essentiels pour la survie et l'adaptation à divers excitants, une exposition prolongée à des taux excessifs de glucocorticoïdes peut être pathogène pour l'organisme.
Le test qui est utilisé ici est le test d'immobilisation décrit par R.M.SAPOLSKY (1983, loc.cit.). Un animal placé pendant 5 minutes dans une enceinte juste suffisante pour le maintenir complètement immobilisé (stress d'immobilisation) développe une réaction d'anxiété qui provoque l'augmentation rapide du taux de corticostérone.
Dans cette expérience, on a utilisé des groupes de 4 à 8 rats Sprague-Dawley jeunes (2 mois) et âgés (20 à 21 mois). Les groupes traités ont reçu 17 mg/kg du composé I ou 5 mg/kg de chlordiazépoxide per os 60 minutes avant le test d'immobilisation. Les groupes témoins n'ont reçu que la solution saline (0,9 % NaCl). Le taux de corticostérone plasmatique est mesuré avant le stress, à la fin du stress et 30 minutes plus tard pour évaluer l'état de récupération des animaux.
Le tableau V donne pour chaque groupe d'animaux la moyenne du taux plasmatique de corticostérone mesuré dans ces conditions.

**TABLEAU V**

| Réponse à un stress d'immobilisation | | | |
|---|---|---|---|
| Animaux/Traitement | Taux de corticostérone plasmatique (ng/ml) | | |
| | basal X ± SEM | à la fin du stress X ± SEM | après 30 minutes X ± SEM |
| Jeunes/Témoins | 19 ± 4 | 1333 ± 98 | 163 ± 10 |
| Agés/Témoins | 238 ± 60 | 1034 ± 94 | 933 ± 145 |
| Agés/Composé I (17 mg/kg) | 83 ± 21* | 1294 ± 101 | 480 ± 54* |
| Agés/CDP (5 mg/kg) | 78 ± 23* | 1533 ± 128* | 1182 ± 185 |
| CDP : chlordiazépoxide | | | |

| | | | |
|---|---|---|---|
| (*) : différence significative par rapport au groupe d'animauxâgés/témoins: P ≤ 0,05, test t de Student. | | | |

Les résultats confirment que, dans les groupes témoins, les rats âgés ont un taux basal de corticostérone beaucoup plus élevé que les rats jeunes. A la fin du stress, ce taux a considérablement augmenté dans les deux cas, mais 30 minutes après la fin du stress, ce taux reste toujours très élevé chez les animaux âgés/témoins. La récupération chez ces animaux est plus lente que chez les rats jeunes.
Dans les groupes d'animaux âgés traités, soit par le composé I ou par le chlordiazépoxide, le taux basal de corticostérone est moins élevé que dans le groupe des animaux témoins âgés, tout en étant cependant supérieur à celui des animaux témoins jeunes.
Le stress augmente aussi considérablement le taux de corticostérone plasmatique des animaux âgés, toutefois le groupe âgé traité par le composé I récupère beaucoup plus rapidement que celui traité par le chlordiazépoxide comme l'indique le taux de corticostérone mesuré après 30 minutes.
Ce résultat montre que, contrairement au chlordiazépoxide, le (S)-(-)-α-éthyl-2-oxo-1-pyrrolidineacétamide exerce une action bénéfique chez les sujets âgés stressés en facilitant l'adaptation physiologique à la situation de stress.
6.- Syndrome de sevrage de benzodiazépines.
L'arrêt brutal d'un traitement thérapeutique prolongé par des benzodiazépines s'accompagne fréquemment d'un syndrome de sevrage caractérisé par un large éventail de symptômes dus à une dépendance physique au médicament, par exemple des tremblements, des nausées, des vertiges ou de l'hypertension pour n'en citer que quelques uns, ainsi que par des symptômes habituels de l'anxiété qui sont intenses chez l'homme (J.H.WOODS et al., Pharmacol.Rev., 39, (1987), 251-419; M.H.LADER, "Abuse Potential, Tolerance and Dependance on Chronic Anxiolytic Treatment" dans "Target Receptors for Anxiolytics and Hypnotics: From Molecular Pharmacology to Therapeutics.", Eds.J.Mendlewicz and G.Racagni, Karger, Basel, Vol. 3, (1992), p.46-54).
En se basant sur l'observation que le sevrage est associé à des stimuli anxiogènes, on a suggéré d'utiliser des modèles animaux d'anxiété pour détecter les signes de sevrage chez l'animal.(M.W.EMMETT-OGLESBY et al., Psychopharmacology, 101, (1990), 292-309).
La réponse anxiogène du sevrage du diazepam, notamment, a fait l'objet d'études sur le rat au moyen du test d'anxiété du labyrinthe en croix surélevé et s'exprime dans ce test par une réduction significative de l'exploration des bras ouverts.(S.E.FILE et al.,Psychopharmacology 105, (1991), 578-582) .
Ce même modèle a été utilisé pour étudier l'effet anxiogène du sevrage du chlordiazépoxide chez la souris et mettre en évidence l'activité anxiolytique du (S)-(-)-α-éthyl-2-oxo-1-pyrrolidineacétamide (composé I) sur ce syndrome.
On a utilisé des groupes de 17 souris (NMRI) répartis comme suit:
- groupe 1:: les animaux sont traités par voie intrapéritonéale, deux fois par jour, pendant 21 jours, avec des doses croissantes de chlordiazépoxide allant de 10 à 40 mg/kg;
la dernière injection a lieu 60 minutes avant le test;
- groupe 2:: le sevrage du chlordiazépoxide a lieu 24 heures avant le test et les animaux sevrés reçoivent une solution physiologique;
- groupe 3:: les animaux sevrés reçoivent le composé I à la dose de 17 mg/kg par voie intrapéritonéale;
- groupe 4:: les animaux sevrés reçoivent le composé I à la dose de 54 mg/kg par voie intrapéritonéale.

Le groupe témoin reçoit seulement 10 ml/kg d'une solution physiologique.
Le tableau VI donne les moyennes du nombre total d'entrées dans les bras du labyrinthe, le pourcentage d'entrées dans les bras ouverts par rapport au nombre total d'entrées ainsi que la moyenne du temps passé à l'exploration des bras ouverts pour chaque groupe d'animaux, témoins et traités.

**TABLEAU VI**

| Syndrome de sevrage de benzodiazépines Test du labyrinthe en croix surélevé | | | |
|---|---|---|---|
| Groupe d'animaux | Nombre total d'entrées X ± SEM | Bras ouverts | |
| | | Nombre d'entrées (%) X ± SEM | Temps (sec) X ± SEM |
| Témoin | 13,1 ± 0,4 | 23,9 ± 1,5 | 26,7 ± 2,3 |
| 1 | 22,9 ± 1,0 | 44,0 ± 3,5 | 72,1 ± 6,0 |
| 2 | 9,8 ± 0,6 | 10,1 ± 2,9 | 8,8 ± 3,0 |
| 3 | 12,5 ± 0,7 | 15,5 ± 2,7 | 16,8 ± 4,1* |
| 4 | 12,2 ± 0,5 | 23,5 ± 3,6* | 27,3 ± 5,4* |

| | | | |
|---|---|---|---|
| (*): augmentation significative par rapport au groupe 2: P ≤ 0,05; test U de Mann-Whitney. | | | |

Les résultats montrent clairement l'activité anxiolytique du chlordiazépoxide sur les animaux traités en chronique par ce médicament (groupe 1) par rapport aux témoins, ce qui se traduit par une augmentation du nombre d'entrées dans les bras ouverts du labyrinthe et du temps consacré à les explorer.
Le sevrage (groupe 2) provoque une anxiété qui se manifeste par une diminution importante de ces entrées, tandis que le (S)-(-)-α-éthyl-2-oxo-1-pyrrolidineacétamide (composé I), à la dose de 54 mg/kg (groupe 4) supprime l'effet anxiogène du sevrage du chlordiazépoxide et rétablit chez les animaux traités un comportement qui est semblable à celui du groupe témoin.
7.- Test du labyrinthe aquatique.
Introduit à l'origine par R.MORRIS (J.Neurosci.Methods, 11, (1984),47-60), le test du labyrinthe aquatique est un test d'apprentissage et de mémorisation dans lequel des rats apprennent à s'échapper de l'eau en nageant vers une plate-forme située en un endroit déterminé dans un grand bassin circulaire rempli d'une eau opaque.
Comme la plate-forme est immergée sous la surface de l'eau, elle est invisible, et l'animal doit apprendre des repères de distances pour retrouver le refuge. Ce test est sensible à l'effet amnésique antérograde de certains médicaments, en particulier des benzodiazépines comme le chlordiazépoxide.
L'apprentissage est exprimé par le temps (en secondes) mis par l'animal pour trouver la plate-forme au cours de séances d'entraînement répétées 4 jours consécutivement. L'effet amnésiant d'un médicament se traduit par une augmentation du temps nécessaire à l'animal pour rejoindre le refuge. (N.Mc NAUGHTON et al., Behav.Brain Res., 24 (1987),39-46).
Dans ce test, on a utilisé des groupes de 8 rats SPRAGUE-DAWLEY. Les animaux traités ont reçu le composé I ou le chlordiazépoxide, par administration orale 60 minutes avant le test. Le groupe témoin n'a reçu que 5 ml/kg d'une solution physiologique.
Le tableau VII donne la moyenne des temps mis par les animaux des groupes traités aux doses indiquées et du groupe témoin, pour retrouver la plate-forme au cours des 4 séances successives. 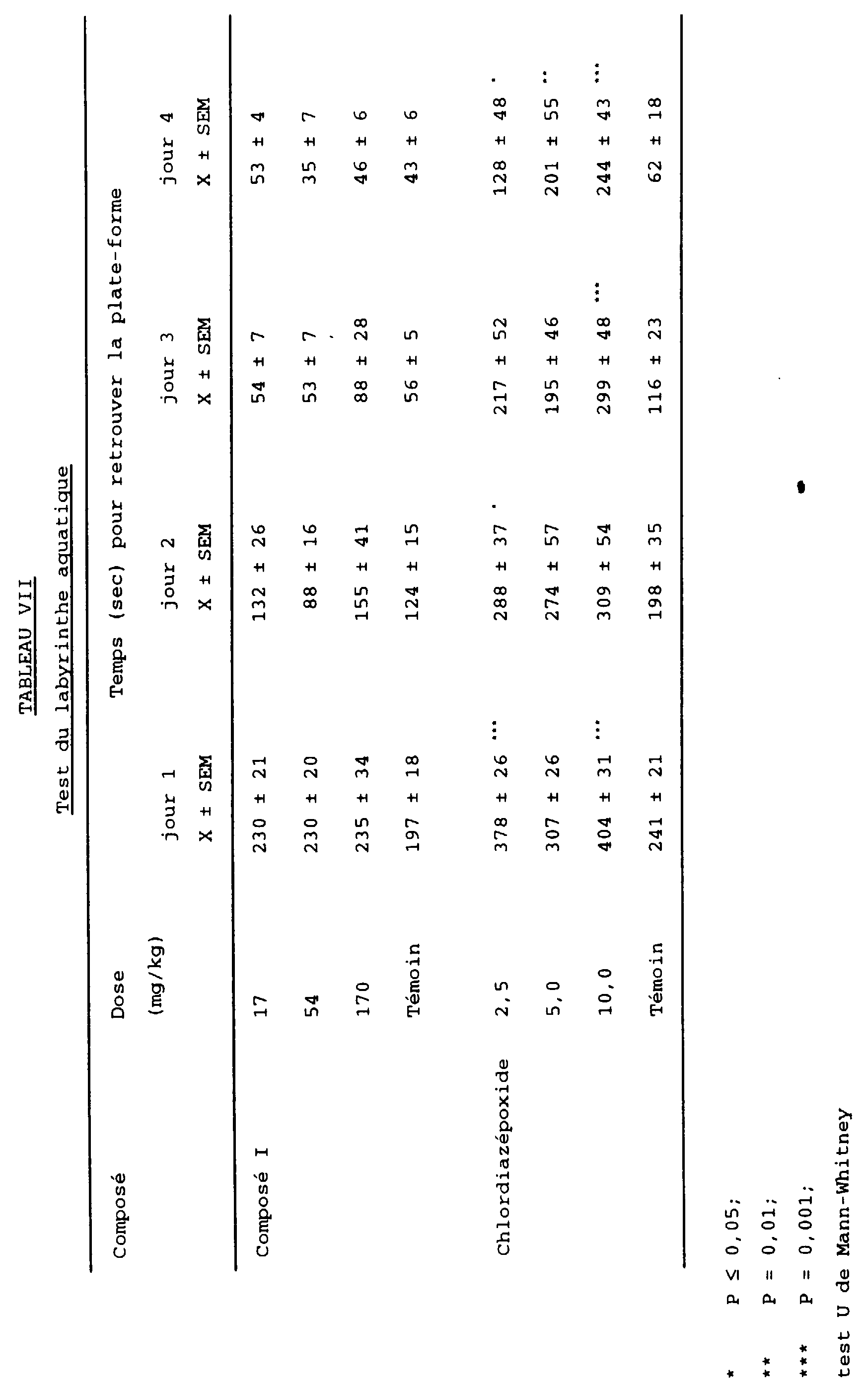
Les résultats montrent que le chlordiazépoxide exerce un effet amnésiant chez l'animal, qui se manifeste par une augmentation significative du temps nécessaire pour retrouver la plate-forme.
Malgré l'apprentissage naturel qui se traduit par une réduction du temps nécessaire au fur et à mesure des séances, l'effet amnésique du chlordiazépoxide chez les animaux traités, par rapport aux animaux témoins, est toujours bien apparent au 4^{ème} jour.
Par contre, le (S)-(-)-α-éthyl-2-oxo-1-pyrrolidineacétamide (composé I), même à la plus forte dose (170 mg/kg), ne produit pas de différence significative du temps nécessaire pour retrouver la plate-forme par rapport au groupe témoin. Il est dépourvu des propriétés amnésiques du chlordiazépoxide qui constituent un effet secondaire non souhaité au cours du traitement de l'anxiété par les benzodiazépines (J.H.WOODS et al., Pharmacol.Rev., 39, (1987), 251-419).
8.- Neurotoxicité
Les effets neurotoxiques du composé I ont été comparés à ceux du diazépam et du chlordiazépoxide au moyen de deux tests pratiqués sur des souris: le test d'Irwin et le test du Rotarod.
- Le test d'Irwin (S.IRWIN, Psychopharmacologia (Berl.) 13, (1968), 222-257) est un procédé d'observation systématique des effets neurotoxiques d'un médicament sur le comportement général et l'état physiologique d'un rongeur, rat ou souris.
   Les changements observés dans différents paramètres du comportement et de l'état de l'animal sont notés et quantifiés au moyen d'une échelle arbitraire de cotations allant de 0 à 8, par rapport aux animaux témoins. Les composés ont été administrés à des souris à doses croissantes par voie intrapéritonéale et les animaux sont observés à 5, 10, 30, 60 et 120 minutes après l'injection.
- Le test du Rotarod consiste à évaluer la capacité d'un rat ou d'une souris à se maintenir en équilibre pendant 60 secondes sur un barreau de 3 cm de diamètre tournant à la vitesse de 6 tours par minute (N.W.DUNHAM et al., J.Am.Pharm.Assoc., XLVI, (1957), 208-209).
   Les composés ont été administrés à des souris à des doses croissantes par voie intrapéritonéale 60 minutes avant les tests.

Le tableau VIII indique, pour le test d'Irwin, les doses (mg/kg) des composés qui provoquent les premiers changements perceptibles dans les paramètres observés. Pour le test du Rotarod, le tableau VIII donne la dose ED₅₀ (mg/kg) des composés testés qui est suffisante pour que 50 % des souris soumises à l'essai soient incapables de se maintenir sur le barreau tournant pendant 60 secondes.

**TABLEAU VIII**

| Neurotoxicité | | | |
|---|---|---|---|
| Paramètres | Composé I (mg/kg) | Diazepam (mg/kg) | Chlordiazépoxide (mg/kg) |
| Test d'Irwin | | | |
| Réduction de l'activité spontanée | NE | 0,9 | 10,8 |
| Réduction de la réactivité à la manipulation | NE | 2,8 | 33,5 |
| Troubles de la vigilance | NE | 2,8 | 33,5 |
| Relâchement musculaire | 540 | 0,9 | 3,4 |
| Incoordination motrice | NE | 2,8 | 10,8 |
| Trouble du réflexe de redressement | 1700 | 2,8 | 6,1 |

| Test du Rotarod | | | |
|---|---|---|---|
| ED₅₀ | > 1700 | 2,8 | 5,7 |
| NE: pas d'effet jusqu'à 1700 mg/kg | | | |

Les résultats montrent que les premiers effets neurotoxiques du (S)-(-)-α-éthyl-2-oxo-1-pyrrolidineacétamide (composé I) n'apparaissent qu'à des doses très élevées. A 540 mg/kg apparaissent les premières manifestations de relâchement musculaire et à 1700 mg/kg, dose testée la plus élevée, une perturbation du réflexe de redressement devient apparente. Cette dernière dose est jusqu'à 100 fois supérieure à la dose anxiolytique.
Le diazépam et le chlordiazépoxide, par contre, réduisent l'activité, la réactivité et la vigilance et produisent du relâchement musculaire et des troubles de la coordination motrice à des doses relativement faibles et qui ne sont que 2 à 3 fois supérieures aux doses anxiolytiques.
Chez l'animal, le composé I présente donc une marge thérapeutique entre la dose anxiolytique et les doses neurotoxiques beaucoup plus élevée que les benzodiazépines.
9.- Essai clinique
L'activité anxiolytique du (S)-(-)-α-éthyl-2-oxo-1-pyrrolidineacétamide a été testée au cours d'un essai clinique ouvert effectué sur 76 patients des deux sexes, d'un âge moyen de 39 ans, et souffrant de troubles d'anxiété divers: phobies, paniques, anxiété généralisée et troubles obsessionnels.
Le composé a été administré à ces patients par voie orale à la dose moyenne de 250 mg, deux fois par jour, pendant quatre semaines.
Le degré d'anxiété des patients a été évalué au moyen de l'échelle d'anxiété d'Hamilton (M.HAMILTON, Brit.J.Med.Psychol., 32, (1959), 50-55). Le composé est considéré comme actif chaque fois que l'on observe une réduction d'au moins 30 % du taux d'anxiété.
Le composé s'est montré bien actif chez 56 patients. Ceux qui répondent le mieux au traitement sont ceux qui souffrent d'anxiété d'origine psychique par rapport à ceux qui sont atteints d'anxiété d'origine psychosociale. De la somnolence a été occasionnellement rapportée chez certains patients, mais suffisamment légère pour ne pas être considérée comme de la sédation. En effet, les tests de capacité mentale, de vigilance et de mémoire n'ont pas été affectés par le traitement.

## Revendications

1. Utilisation du (S)-(-)-α-éthyl-2-oxo-1-pyrrolidineacétamide de formule 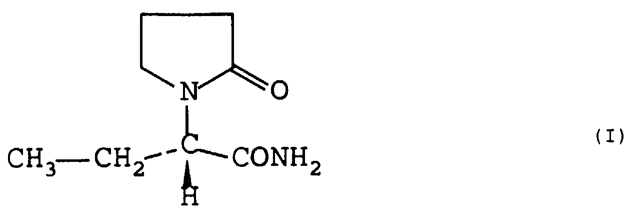 pour la préparation de médicaments destinés au traitement de l'anxiété.

2. Utilisation du (S)-(-)-α-éthyl-2-oxo-1-pyrrolidineacétamide selon la revendication 1 pour la préparation de médicaments destinés au traitement des états d'anxiété provoqués par un stress aigu post-traumatique.

3. Utilisation du (S)-(-)-α-éthyl-2-oxo-1-pyrrolidineacétamide selon la revendication 1 pour la préparation de médicaments destinés au traitement de l'anxiété induite par le sevrage d'une administration chronique de benzodiazépines.

4. Utilisation selon l'une quelconque des revendications 1 à 3 caractérisée en ce que la quantité thérapeutiquement efficace du composé de formule I est comprise entre 5 et 70 mg/kg par jour.

## Patentansprüche

1. Verwendung von (S)-(-)-α-Ethyl-2-oxo-1-pyrrolidinacetamid der Formel 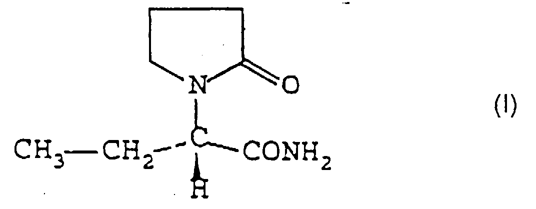 zur Herstellung von Medikamenten, die für die Behandlung der Angstzustände bestimmt sind.

2. Verwendung von (S)-(-)-α-Ethyl-2-oxo-1-pyrrolidinacetamid gemäß Anspruch 1 zur Herstellung von Medikamenten, die für die Behandlung der Angstzustände, die durch einen akuten posttraumatischen Streß hervorgerufen werden, bestimmt sind.

3. Verwendung von (S)-(-)-α-Ethyl-2-oxo-1-pyrrolidinacetamid gemäß Anspruch 1 zur Herstellung von Medikamenten, die für die Behandlung der Angstzustände, die durch das Absetzen einer chronischen Verabreichung von Benzodiazepinen hervorgerufen werden, bestimmt sind.

4. Verwendung gemäß einem der Ansprüche 1 bis 3, dadurch gekennzeichnet, dass die therapeutisch wirksame Menge der Verbindung der Formel I zwischen 5 und 70 mg/kg pro Tag liegt.

## Claims

1. Use of (S)-(-)-α-ethyl-2-oxo-1-pyrrolidineacetamide of formula 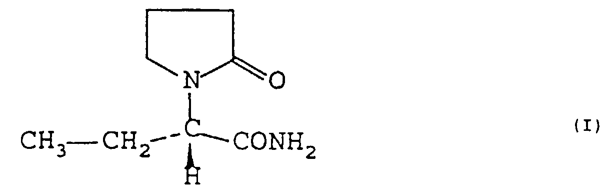 for the preparation of drugs intended for the treatment of anxiety.

2. Use of (S)-(-)-α-ethyl-2-oxo-1-pyrrolidineacetamide according to claim 1 for the preparation of drugs intended for the treatment of anxiety states caused by acute post-traumatic stress.

3. Use of (S)-(-)-α-ethyl-2-oxo-1-pyrrolidineacetamide according to claim 1 for the preparation of drugs intended for the treatment of anxiety induced by the withdrawal of the chronic administration of benzodiazepines.

4. Use according to any one of claims 1 to 3, characterized in that the effective therapeutic quantity of the compound of formula I is comprised between 5 and 70 mg/kg per day.
